# EUROPÄISCHE PATENTSCHRIFT

(11) **EP 2 433 135 B1**
(45) Veröffentlichungstag und Bekanntmachung des Hinweises auf die Patenterteilung: **14.08.2013**
(21) Anmeldenummer: 10720707.8
(22) Anmeldetag: 19.05.2010
(51) Int. Cl.: G01N 33/574, G01N 33/58

(54) **VERFAHREN ZUR QUALITATIVEN UND/ODER QUANTITATIVEN BESTIMMUNG VON TUMORZELLEN**
METHOD FOR THE QUALITATIVE AND/OR QUANTITATIVE ANALYSIS OF TUMOUR CELLS
PROCÉDÉ DE DÉTERMINATION QUALITATIVE ET/OU QUANTITATIVE DE CELLULES TUMORALES

(30) Priorität: 19.05.2009 AT 7842009
(43) Veröffentlichungstag der Anmeldung: 28.03.2012
(73) Patentinhaber: Onkotec GmbH, 3830 Waidhofen/Thaya (AT)
(72) Erfinder: GVICHIYA, Khatuna, Elizbarowna, St. Petersburg 198035 (RU); EDETSBERGER, Michael, A-1140 Wien (AT); KNAPP, Martin, A-1170 Wien (AT)
(74) Vertreter: Schober, Elisabeth
(86) Internationale Anmeldenummer: PCT/AT2010/000169
(87) Internationale Veröffentlichungsnummer: WO 2010/132908

(56) Entgegenhaltungen:
- WO-A1-00/53170
- CHAKRABORTY A ET AL: "Photoinduced intermolecular electron transfer from dimethyl aniline to 7-amino Coumarin dyes in the surface of beta-cyclodextrin" SPECTROCHIMICA ACTA. PART A: MOLECULAR AND BIOMOLECULAR SPECTROSCOPY, ELSEVIER, AMSTERDAM, NL LNKD- DOI:10.1016/J.SAA.2005.08.007, Bd. 64, Nr. 3, 1. Juni 2006 (2006-06-01), Seiten 801-808, XP025176730 ISSN: 1386-1425 [gefunden am 2006-06-01]
- TWEEDELL K S: "LOCALIZATION OF GLYCO CONJUGATES ON THE SURFACES OF PRO NEPHRIC TUMOR CELLS IN-VITRO" HISTOCHEMISTRY, Bd. 75, Nr. 4, 1982, Seiten 507-522, XP009135753 ISSN: 0301-5564
- PUN S H ET AL: "TARGETED DELIVERY OF RNA-CLEAVING DNA ENZYME (DNAZYME) TO TUMOR TISSUE BY TRANSFERRIN-MODIFIED, CYCLODEXTRIN-BASED PARTICLES" CANCER BIOLOGY AND THERAPY, LANDES BIOSCIENCE, US, Bd. 3, Nr. 7, 1. Juli 2004 (2004-07-01), Seiten 641-650, XP008054270 ISSN: 1538-4047

## Beschreibung

Die Erfindung betrifft ein Verfahren und Mittel zur Markierung von Tumorzellen. Insbesondere betrifft die Erfindung ein Verfahren und Mittel zur qualitativen und quantitativen Bestimmung von Tumorzellen.

Bei entdifferenzierten Tumorzellen funktioniert die klassische Zytologie sehr gut. Ausdifferenzierte Tumorzellen können dagegen mittels klassischer zytologischer Verfahren nur sehr schwer von gesunden Zellen unterschieden werden. Es besteht daher ein Bedarf an Verfahren und Mittel, welche in der Lage sind, ausdifferenzierte Tumorzellen in Gegenwart von normalen Zellen, insbesondere in Körperflüssigkeiten, qualitativ und/oder quantitativ zu bestimmen.

Es besteht kein Zweifel, dass fluoreszierende organische Substanzen eine wichtige Rolle in Zukunftstechnologien, wie Biosensoren, Photokatalysatoren oder Optoelektronik spielen werden bzw. bereits spielen. Speziell in der Gruppe der Amino-coumarine gibt es einige Vertreter, die aufgrund ihrer photophysikalischen Eigenschaften, wie Photostabilität und optimaler Quantenausbeute, in vielen Bereichen erfolgreich eingesetzt werden [1-4].

Der Einsatz von Aminocoumarinen in Nanopartikeln ist ebenso bekannt, dabei dient das Aminocoumarin aber ausschließlich zur Markierung und Charakterisierung der Stabilität von Nanopartikel und nicht zur direkten fluoreszenten Markierung von Zellen [5], da die Fluoreszenzmarkierung indirekt von der Aufnahme der Nanopartikel abhängig ist. In den beschriebenen Zellen dient der Farbstoff zur selektiven Markierung der Nanopartikel und man untersucht die Aufnahmemechanismen bzw. die Interaktionen zwischen Nanopartikel und Tumorzellen.

Bei den Aminocoumarinen handelt es sich um organische Substanzen, die in wässriger Umgebung eine sehr geringe Löslichkeit besitzen bzw. nahezu unlöslich sind [6,7].

Allerdings ist bekannt, dass Coumarin-6 sowohl in wässriger als auch in abgepufferter Umgebung durch Cyclodextrine (auch als Schardinger-Dextrine bekannte Cycloamylosen oder Cycloglucane bzw. cyclische Oligosaccharide) in Lösung gehalten werden kann, da Cyclodextrine mit anderen Molekülen geeigneter Größe und Polarität Einschlusskomplexe bilden können [7,8, 13]. Dabei sind speziell die hydrophilen Cyclodextrine in der Lage, die Abgaberate und das Zeitprofil der Substanzfreisetzung zu beeinflussen [9]. Cyclodextrine wurden auch sehr erfolgreich als Stabilisatoren für Proteine und Peptide eingesetzt, diese Stabilisierung wird durch die Interaktion zwischen den Proteinen oder Peptiden und den hydrophilen Bereichen und hydrophoben Cavities der Cyclodextrine erreicht [8].

In den letzten Jahrzehnten entwickelte sich die selektive und spezifische Photodiagnostik bzw. Therapie von malignem Gewebe zu einem eigenständigen medizinischen Fachgebiet [10, 11], wobei in erster Linie die Ausnutzung von photophysikalischen und photodynamischen Effekten zur Diagnostik eingesetzt wird. Es ist auch bekannt, dass verschiedene Coumarine und Coumarin-assoziierte Produkte Gegenstand verschiedenere Studien zur Abschätzung der potentiellen therapeutischen Möglichkeiten zur Krebstherapie sind [12].

Das Coumarin-6, ein grün fluoreszierender Vertreter der nahezu wasserunlöslichen 7-Amino-coumarine, wird schon seit einiger Zeit, aufgrund seiner optimalen photophysikalischen Eigenschaften, als Laser-Referenz-Substanz eingesetzt [14]. Durch die Komplexbildung zwischen dem Coumarin-6 und Cyclodextrinen (speziell beta- und gamma-Cyclodextrin) können grün fluoreszierende Einschlusskomplexe hergestellt werden, die gut wasser- bzw. pufferlöslich sind, sodass auch wässrige Lösungen mit höherer Coumarin-6-Konzentration hergestellt werden können. Dabei können mit den Einschlusskomplexen wässrige Coumarin-6-Lösungen zubereitet werden, die eine bis zu 1000-fach höhere Coumarin-6-Konzentration im Vergleich zu wässrigen Lösungen von reinem Coumarin-6 aufweisen. In gleicher Weise können durch die Bildung von Einschlusskomplexen auch wässrige Lösungen anderer Coumarine mit höherer Coumarin-Konzentration hergestellt werden.

WO 00/53170 beschreibt die Verwendung von Hypericin zur Diagnose von Karzinomen. Dabei wird ein Farbstoffkomplex aus Hypericin und Cyclodextrin verwendet.

Die Aufgabe der vorliegenden Erfindung besteht darin, ein Verfahren und Mittel bereitzustellen, die es erlauben, ausdifferenzierte Tumorzellen, gegebenenfalls direkt in Körperflüssigkeiten, zu markieren und in der Folge sowohl qualitativ als auch quantitativ zu bestimmen.

Diese Aufgabe wird erfindungsgemäß durch ein Verfahren, umfassend die Schritte:
a) Erzeugen eines Farbstoffkomplexes aus einem AminoCoumarin und Cyclodextrin;
b) Versetzen der Tumorzellen mit dem in Schritt a) hergestellten Farbstoffkomplex;
c) Inkubation der Zellen mit dem in Schritt a) hergestellten Farbstoffkomplex;
d) Bestimmen der Zellen mittels fluoreszensspektrometrischer Messung und/oder fluoreszenzmikroskopischer Methoden;
gelöst.

Die Vorteile des erfindungsgemäßen Verfahren bestehen u.a. darin, dass durch den Einsatz von wasserlöslichen Komplexen aus Aminocoumarinen und Cyclodextrinen die Anwendung von Zell- oder Gewebe-irritierenden organischen Lösungsmitteln vermieden und eine physiologisch günstige Form zur Diagnostik von Tumorzellen möglich wird.

Die Farbstoffkomplexe aus Aminocoumarinen und Cyclodextrinen dienen im erfindungsgemäßen Verfahren der ex-vivo Markierung von Zellen, die aufgrund einer Läsion in die Umgebungsflüssigkeit abgeben werden. Die selektive Anreicherung des Farbstoffkomplexes erfolgt aufgrund verschiedener, morphologischer Unterschiede von Tumorzellen gegenüber gesunden Zellen, da sowohl gutartige als auch bösartige Veränderungen mit einem veränderten Zellstoffwechsel verbunden sind.

Da ausdifferenzierte Tumorzellen bereits fluoreszenzpositiv sind, können mit dem erfindungsgemäßen Verfahren solche Zellen in Gegenwart von gesunden Zellen, oder Zellbestandteilen, insbesondere auch in Körperflüssigkeiten, spezifisch markiert und in der Folge detektiert werden. Somit ist das erfindungsgemäße Verfahren in der Lage, Tumorzellen eindeutig zu markieren, wobei in den Proben vorhandene Blutzellen, wie Erythrozyten, keine nennenswerten Hintergrund- oder Störsignale liefern. Die Markierung und/oder Detektion von Tumorzellen kann mittels des erfindungsgemäßen Verfahrens an abgetrennten (isolierten), aufkonzentrierten oder suspendierten Tumorzellen, aber auch direkt an Tumorzellen in den Körperflüssigkeiten bei unterschiedlichen pH-Werten erfolgen. Die pH-Werte können dabei von pH 2 bis pH 11, bevorzugt von pH 5 bis pH 8,5, wie beispielsweise im Harn, ferner bevorzugt pH 6,5 bis pH 7,5, wie beispielsweise im Blut, reichen. Eine Bestimmung von Tumorzellen in Sputum und anderen Körperflüssigkeiten ist ebenfalls möglich.

Da im erfindungsgemäßen Verfahren nahezu keine Wechselwirkungen mit gesunden Zellen, Zellbestandteilen oder der Suspensionslösung (Wasser, Puffer, Harn, etc.) auftreten, muss aufgrund dieser hohen Spezifizität gegenüber Tumorzellen, der Farbstoffkomplex für die Detektion/Auswertung auch nicht entfernt werden.

Nach erfolgter Bindung des Farbstoffkomplexes an die Tumorzellen können die so markierten Zellen mittels Fluoreszenzmikroskopie und/oder -Spektroskopie detektiert, unterschieden und analysiert werden.

In zellbiologischen Untersuchungsreihen konnte gezeigt werden, dass das erfindungemäße Verfahren bevorzugt maligne oder pathologisch auffällige Zellen markiert, wobei es auch bei verschiedenen Ausdifferenzierungsgraden der gleichen Tumorzellen bzw. unterschiedlichen Tumorzellen zu unterscheidbaren Anlagerungsmustern des Farbstoffkomplexes kommt.

So konnte gezeigt werden, dass bei urothelialen Tumorzellen bevorzugt bestimmte, "Mikrovilli"-ähnliche Strukturen an der Zellmembran angefärbt werden, welche sich durch bläschen-ähnliche Strukturen an der Außenseite der Zellen manifestieren. Im Gegensatz zu den Urothelialzellen, findet sich bei tumorgenen Fibroblasten nur eine intrazelluläre Anhäufung ohne starke Interaktion mit der Zellmembran.

Weiters konnte gezeigt werden, dass sich das Verteilungsmuster des Farbstoffkomplexes bei unterschiedlichen Ausdifferenzierungsgraden der Tumorzellen verändert. So findet sich beispielsweise in Zellen des Ausdifferenzierungsgrades II eine punktuelle Anhäufung des Farbstoffes im Inneren der Zelle, die bei Grad-IV Zellen nicht so ausgeprägt ist.

Dies stellt einen weiteren Vorteil des erfindungsgemäßen Verfahrens dar, da bei alleiniger Verwendung des Aminocoumarin-Farbstoffes, d.h. in Abwesenheit von Cyclodextrinen, in wässrig/ethanolischer Lösung nur eine geringere Anfärbung und keine deutlichen Unterschiede im Verteilungsmuster der mit Farbstoff markierten Tumorzellen auftreten, wodurch eine Unterscheidung zwischen den einzelnen Ausdifferenzierungsgraden nicht möglich ist.

Es wurde festgestellt, dass eine Reihe verschiedener Aminocoumarine mit Cyclodextrinen Farbstoffkomplexe bilden, die erfindungsgemäß eingesetzt werden können.

Die für das erfindungsgemäße Verfahren einsetzbaren Amino-coumarine umfassen Coumarin-6 (3-(2-Benzothiazolyl)-7-(di-ethylamino)coumarin), Coumarin-30 (auch als Coumarin 515 bezeichnet; 7-(Diethylamino)-3-(1-methyl-1H-benzo[d]imidazol-2-yl)-2H-chromen-2-on), Coumarin-35 (7-Diethylamino-4-trifluormethyl-coumarin), Coumarin-47 (7-Diethylamino-4-methyl-chromen-2-on), Coumarin-102 (auch als Coumarin-480 bezeichnet; 2,3,6,7-Tetrahydro-9-methyl-1H,5H-quinolizino(9,1-gh)coumarin oder 8-Methyl-2,3,5,6-1H,4H-tetrahydroquinolizino[9,9a,1-gh]coumarin), Coumarin-120 (7-Amino-4-methylcoumarin), Coumarin-138 (8-(Dimethylamino)cyclopenta[c]chromen-4(3aH)-on), Coumarin-151 (7-Amino-4-trifluor-methylcoumarin), Coumarin-152 (7-Dimethylamino-4-trifluor-methylcoumarin und Coumarin-153 (2,3,6,7-Tetrahydro-9-trifluormethyl-1H,5H,11H-(1)benzopyranol[6,7,8-ij]-quinolizin-11-on), Coumarin-500 (7-(Ethylamino)-4-(trifluormethyl)-2H-chromen-2-on).

In einer besonders bevorzugten Ausführungsform der Erfindung wird Coumarin-6, d.h. 3-(2-Benzothiazolyl)-7-(diethyl-amino)coumarin eingesetzt.

Zur Verwendung in der vorliegenden Erfindung speziell geeignete Cyclodextrine sind alpha-Cyclodextrin mit 6 alpha-1,4-verbundenen alpha-D-Glycopyranose-Einheiten; beta-Cyclodextrin mit 7 alpha-1,4-verbundenen alpha-D-Glycopyranose-Einheiten; und gamma-Cyclodextrin mit 8 oder mehr alpha-1,4-verbundenen alpha-D-Glycopyranose-Einheiten. Bevorzugt werden beta- und/oder gamma-Cyclodextrin zur Bildung der Farbstoffkomplexe verwendet.

Die Reaktion zum Farbstoffkomplex erfolgt vorzugsweise in wässriger oder gepufferter Lösung bei pH 2 bis pH 11, bevorzugt pH 5 bis pH 8,5, weiter bevorzugt pH 6,5 bis pH 7,5, indem eine alkoholische Lösung des Aminocoumarins und eine wässrige Lösung des Cyclodextrins in einem molaren Verhältnis von Aminocoumarin zu Cyclodextrin von 1:2000 bis 1:20000 gemischt werden und der wasserlösliche Farbstoffkomplex bei Umgebungstemperatur gebildet wird.

In einer bevorzugten Ausführungsform der vorliegenden Erfindung beträgt das molare Verhältnis von Aminocoumarin zu Cyclodextrin 1:10000.

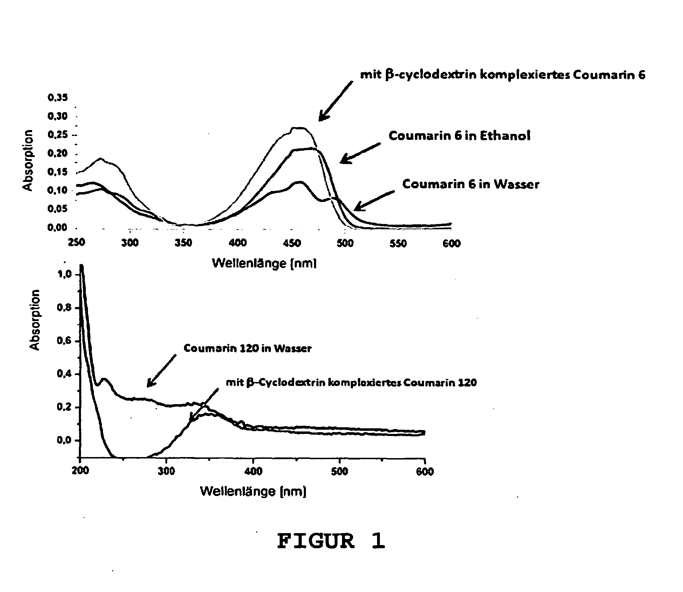

Als wichtigste chemische Charakterisierung des Komplexierungsgrades kann man das Auftreten von zusätzlichen, im Falle von Coumarin 6 rotverschobenen Absorptionspeaks (Abbildung 1 oben) und im Falle von Coumarin 120 (Abbildung 1 unten) blauverschobenen Absorptionspeaks, sofern sich die Substanz in wässriger Umgebung befindet, beobachten. Befinden sich die Coumarine in ethanolischer Umgebung oder werden durch Cyclodextrin in wässriger Umgebung gehalten, treten diese zusätzlichen Peaks nicht auf, denn die Substanzen sind mehrheitlich als Monomere vorliegend und nicht mehrheitlich als Aggregate, wie es normalerweise in wässriger Umgebung der Fall ist. Dieser Effekt ist in erster Linie durch hydrophobe Wechselwirkungen bzw. aromatisches "stacking" erklärbar. Wird die Substanz in einem geeigneten Lösungsmittel gelöst oder mittels Cyclodextrin komplexiert, werden die hydrophoben/aromatischen Interaktionen auf ein Minimum reduziert.

Es wurde festgestellt, dass die Färbespezifität der Farbstoffkomplexe nach einigen Tagen abnimmt, wenn diese in wässriger oder abgepufferter Lösung aufbewahrt werden. So zeigt sich beispielsweise nach einer Woche ein verändertes Färbebild der Tumorzellen, wenn die Farbstoffkomplexlösung in flüssiger Phase bei 4°C gelagert wird. Daher werden die Farbstoffkomplexe in einer bevorzugten Ausführungsform der vorliegenden Erfindung, wenn keine sofortige Verwendung beabsichtigt ist, als Lyophilisate eingesetzt. Wie festgestellt werden konnte, zeigt das Lyophilisat des Fabstoffkomplexes selbst nach zweiwöchiger Lagerung bei Umgebungstemperatur ein gleichbleibendes Färbebild. Dabei kann das Lyophilisat in Tumorzellen enthaltender Kochsalzlösung oder Puffer bei pH 2 bis pH 11, bevorzugt pH 5 bis pH 8,5, weiter bevorzugt pH 6,5 bis pH 7,5, oder bevorzugt in Tumorzellen enthaltenden Körperflüssigkeiten wie Urin, Sputum, oder Blut bei pH 2 bis pH 11, bevorzugt pH 5 bis pH 8,5, weiter bevorzugt pH 6,5 bis pH 7,5, problemlos resolubilisiert werden. In einer weiteren bevorzugten Ausführungsform der Erfindung kann das Lyophilisat auch auf einem festen Träger aufgebracht sein.

Die in der vorliegenden Erfindung verwendeten niedrigen Konzentrationen des Farbstoffkomplexes (10nM Coumarin) und die kurzen Inkubationszeiten von 10 Minuten oder weniger verstärken die Affinität des Farbstoffkomplexes zu Tumorzellen, wobei hier ein unterschiedlicher Cholesterolgehalt zwischen normaler und maligner Zelle eine Rolle zu spielen scheint. Diese kurzen Inkubationszeiten werden in vorteilhafter Weise bei 5°C bis 45°C, bevorzugt bei Umgebungstemperatur von 18°C bis 22°C, insbesondere bevorzugt bei 20°C, oder Körpertemperatur von 35°C bis 39°C, insbesondere bevorzugt bei 37°C erreicht.

Zur Markierung und/oder Detektion mittels des erfindungsgemäßen Verfahrens können die Tumorzellen abgetrennt (isoliert), aufkonzentriert oder suspendiert vorliegen, bevorzugt können die Tumorzellen aber auch direkt in den Körperflüssigkeiten vorliegen. Gewünschtenfalls kann daher im erfindungsgemäßen Verfahren ein Sedimentationsschritt der Tumorzellen vorgesehen werden.

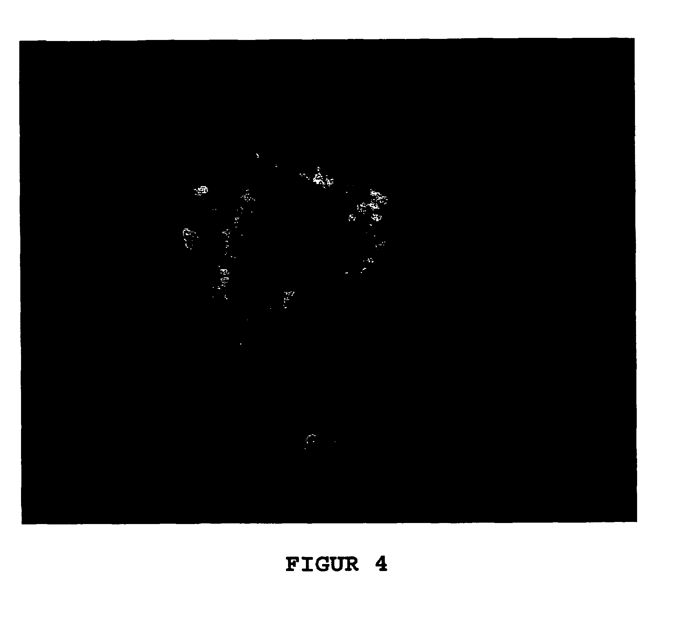

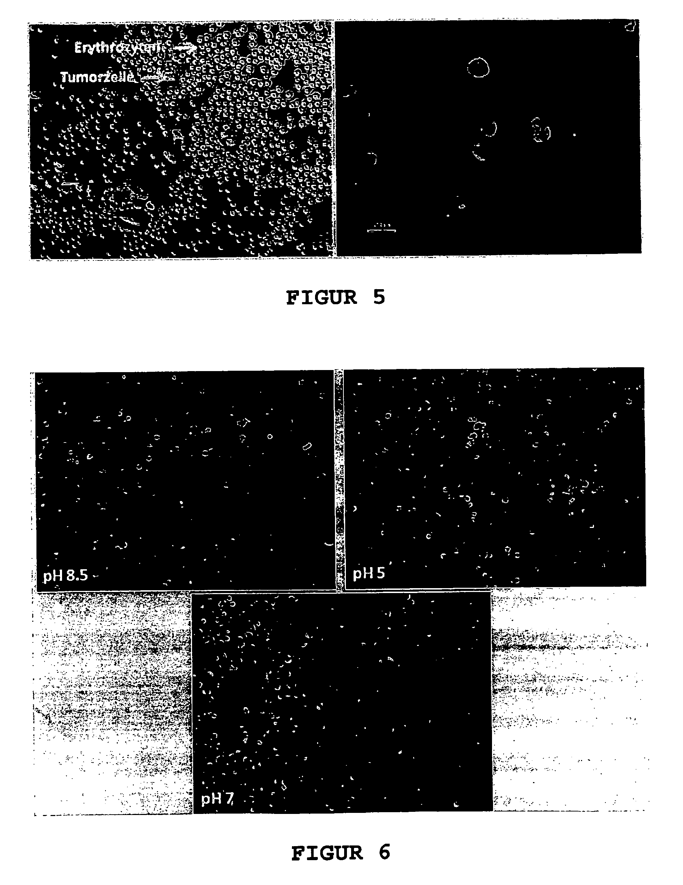

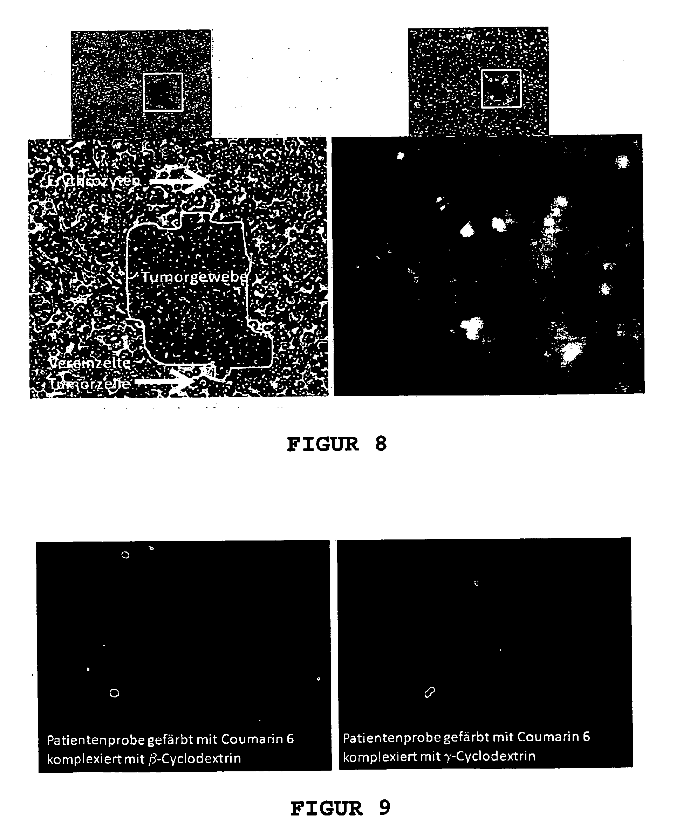

Die Erfindung wird nachstehend anhand der Figuren 1 bis 9 sowie der angeschlossenen Beispiele weiter erläutert.
Figur 1 zeigt die Absorptionsspektren von Coumarin-6 (komplexiert/unkomplexiert) und Coumarin 120 (komplexiert/unkomplexiert) in verschiedenen wässrigen und ethanolischen Umgebungen.

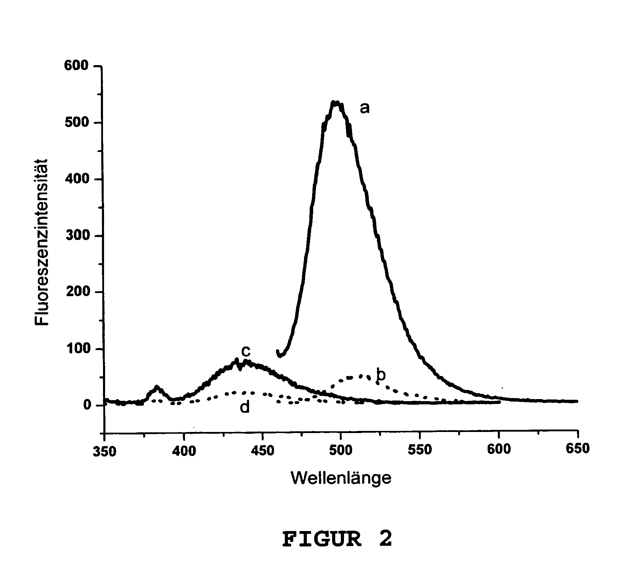

Figur 2 zeigt die fluorimetrische Analyse von Human-Urothelialtumorzellen, Grad-IV in PBS (phospate buffered saline) mit pH 7,4, die mit Coumarin-120- und Coumarin-6-beta-Cyclodextrin-Farbstoffkomplex inkubiert wurden im Vergleich zur Hintergrundfluoreszenz von Coumarin-6 bzw. Coumarin-120 in beta Cyclodextrin. Deutlich ist die 6-fach stärkere Fluoreszenzintensität von Coumarin-6 (a) im Vergleich mit Coumarin-120 (c) erkennbar, sofern die Zellen mit den Farbstoffkomplexen inkubiert wurden. Trotzdem ist eine eindeutige Fluoreszenz der Coumarin-120-beta-Cyclodextrin markierten Zellen erkennbar. Im Vergleich dazu beträgt die Hintergrundfluoreszenz des Coumarin-6 Farbstoffkomplexes (b) rund 10% und des Coumarin-120 Farbstoffkomplexes (d) weniger als 30%.

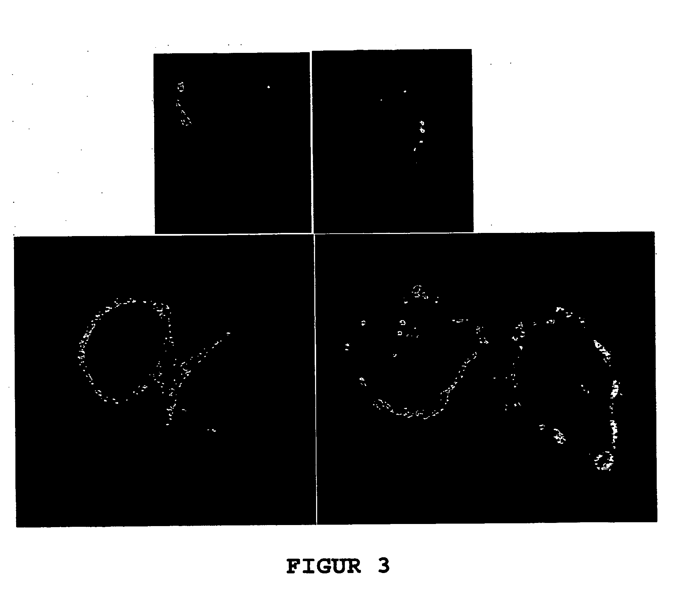

Figur 3 zeigt Human-Urothelialtumorzellen, Grad-IV (oben/ unten links), und Grad-II (oben/unten rechts) (alle in PBS mit pH 7,4), inkubiert mit Coumarin-6-beta-Cyclodextrin-Komplex (unten) und Coumarin-6 in wässrig/ethanolischer Lösung (jeweils oben). Deutlich ist die starke Interaktion zwischen Urothelialzellmembranen und dem Farbstoffkomplex zu sehen. Zusätzlich findet sich ein unterschiedliches, intrazelluläres Verteilungsmuster zwischen den verschiedenen Ausdifferenzierungsgraden. Das Interaktions- und Verteilungsmuster der Urothelialzellen, welche mit dem Farbstoffkomplex gefärbt wurden, unterscheidet sich grundlegend von dem Intensitätsmuster der Zellen, welche mit der reinen ethanolisch/wässrigen Coumarin-6-Lösung gefärbt wurden.
Figur 4 zeigt Tumor-Fibroblasten (in PBS mit pH 7,4), inkubiert mit Coumarin-6-beta-Cyclodextrin-Komplex. Deutlich ist die schwache Interaktion zwischen Zellmembran und dem Farbstoffkomplex zu sehen. Allerdings findet sich eine extrem hohe Farbstoffkonzentration im Zellinneren.
Figur 5 zeigt Human-Urothelialtumorzellen, Grad-IV, (in PBS mit pH 7,4), welche mit menschlichen Erythrozyten gemischt sind. Im Durchlicht (links) können beide Zellarten deutlich unterschieden werden. In der Fluoreszenzaufnahme (rechts) sind nur die gefärbten Tumorzellen erkennbar.
Figur 6 zeigt Human-Urothelialtumorzellen, inkubiert mit Coumarin-6-beta-Cyclodextrin-Komplex, in Harnlösungen mit pH-Werten von 5, 7 und 8,5. In allen Umgebungen ist eine gleichmäßige Anfärbung der Zellen erkennbar und es gibt keine nennenswerte Interaktion zwischen Farbstoffkomplex und dem im Harn befindlichen Protein.

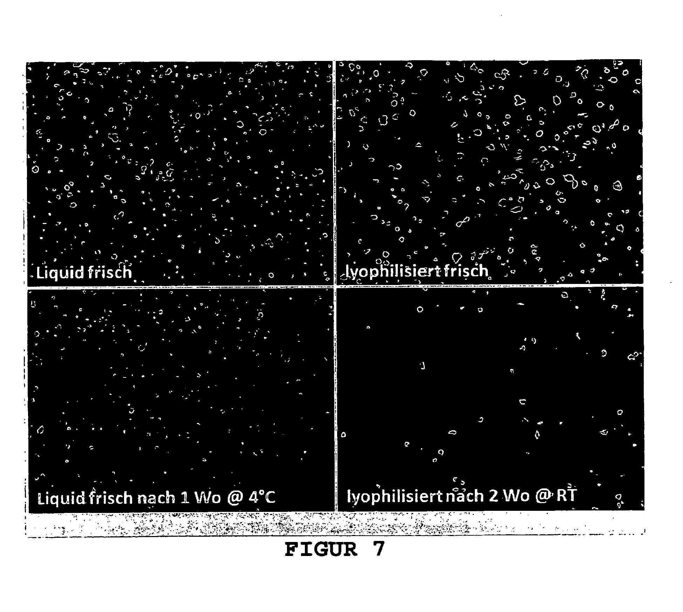

Figur 7 zeigt Human-Urothelialtumorzellen, Grad-IV, (in PBS mit pH 7,4), gefärbt mit einer frisch zubereiteten Coumarin-6-beta-Cyclodextrin-Lösung (oben/links) und mit der gleichen Lösung nach 1-wöchiger Lagerung bei 4°C (unten/links). Im Vergleich dazu wird die gleiche Zelllinie gefärbt durch Zugabe von frisch lyophilisierten Coumarin-6-beta-Cyclodextrin-Komplex zur Tumorzellen enthaltenden Zellsuspension (oben/ rechts) und durch Zugabe von lyophilisierten Coumarin-6-beta-Cyclodextrin-Komplex, nach 2-wöchiger Lagerung bei Umgebungstemperatur, zur Tumorzellen enthaltenden Zellsuspension (unten/rechts).
Figur 8 zeigt eine Patientenprobe (Blasenlavage) inkubiert mit Coumarin-6-beta-Cyclodextrin-Komplex. Deutlich ist der Intensitätsunterschied zwischen Tumorzelle (hell) bzw. Tumorgewebe und Erythrozyten ersichtlich.
Figur 9 zeigt eine Patientenprobe (Blasenlavage) inkubiert mit Coumarin-6-beta-Cyclodextrin-Komplex. (links) und Coumarin-6-gamma-Cyclodextrin-Komplex ((rechts) Deutlich ist zu erkennen, dass es keinen Unterschied in der Färbeintensität gibt.

### Beispiel 1 (Direktfärbung mit flüssiger Färbelösung - Coumarin-6 und beta-Cyclodextrin)

100mg beta-Cyclodextrin (Sigma-Aldrich, Österreich) werden im Ultraschallbad unter Erwärmen auf 65°C in 10ml Wasser gelöst. Zu 10ml dieser Lösung werden unter Rühren 0,5ml einer 1:1000 Verdünnung (in Ethanol) von 35mg 3-(2-Benzothiazolyl)-7-(diethylamino)coumarin in 5ml Ethanol zugefügt, sodass ein Molverhältnis von 3-(2-Benzothiazolyl)-7-(diethylamino)coumarin zu beta-Cyclodextrin von 1:10000 erhalten wird. Die so erhaltene Farbstoffkomplexlösung, welche eine Finalkonzentration von 1µM Coumarin enthält, wird zum Markieren der Tumorzellen verwendet.

Zu 1ml einer Tumorzellen enthaltenden Probe werden 0,01ml der Frischen Farbstoffkomplexlösung zugesetzt. Nach einer Inkubation bei Umgebungstemperatur während 10min werden die Proben spektroskopisch bzw. Fluoreszenzmikroskopisch untersucht und ausgewertet

### Beispiel 2 (Direktfärbung mit flüssiger Färbelösung - Coumarin-6 und gamma-Cyclodextrin)

110mg gamma-Cyclodextrin (Sigma-Aldrich, Österreich) werden in 10ml Wasser bei Umgebungstemperatur gelöst

Zu 10ml dieser Lösung werden unter Rühren 0,5ml einer 1:1000 Verdünnung (in Ethanol) von 35mg 3-(2-Benzothiazolyl)-7-(diethylamino)coumarin in 5ml Ethanol zugefügt, sodass ein Molverhältnis von 3-(2-Benzothiazolyl)-7-(diethylamino)coumarin zu gamma-Cyclodextrin von 1:10000 erhalten wird. Die so erhaltene Farbstoffkomplexlösung, welche eine Finalkonzentration von 1µM Coumarin enthält, wird zum Markieren der Tumorzellen verwendet.

Zu 1ml einer Tumorzellen enthaltenden Probe werden 0.01ml der Frischen Farbstoffkomplexlösung zugesetzt. Nach einer Inkubation bei Umgebungstemperatur während 10min werden die Proben spektroskopisch bzw. Fluoreszenzmikroskopisch untersucht und ausgewertet

### Beispiel 3 (Färbung von Tumorzellen mittels Lyophilisat - Coumarin-6 und beta-Cyclodextrin)

100mg beta-Cyclodextrin (Sigma-Aldrich, Österreich) werden im Ultraschallbad unter Erwärmen auf 65°C in 10ml Wasser gelöst.

Zu 10ml dieser Lösung werden unter Rühren 0,5ml einer 1:1000 Verdünnung (in Ethanol) von 35mg 3-(2-Benzothiazolyl)-7-(diethylamino)coumarin in 5ml Ethanol zugefügt, sodass ein Molverhältnis von 3-(2-Benzothiazolyl)-7-(diethylamino)coumarin zu beta-Cyclodextrin von 1:10000 erhalten wird. Die so erhaltene Farbstoffkomplexlösung wird anschließend in ein Reaktionsgefäß transferiert und bis zur Trockenen lyophilisiert. Das Lyophilisat besitzt eine Finalkonzentration von 1µM an Coumarin.

Zu 10ml einer Tumorzellen enthaltenden Probe werden 1mg des Lyophilisats zugesetzt. Nach einer Inkubation bei Umgebungstemperatur während 10min werden die Proben spektroskopisch bzw. Fluoreszenzmikroskopisch analysiert und ausgewertet

### Beispiel 4 (Färbung von Tumorzellen mittels Lyophilisat - Coumarin-6 und gamma-Cyclodextrin)

110mg gamma-Cyclodextrin (Sigma-Aldrich, Österreich) werden in 10ml Wasser bei Umgebungstemperatur gelöst.

Zu 10ml dieser Lösung werden unter Rühren 0,5ml einer 1:1000 Verdünnung (in Ethanol) von 35mg 3-(2-Benzothiazolyl)-7-(diethylamino)coumarin in 5ml Ethanol zugefügt, sodass ein Molverhältnis von 3-(2-Benzothiazolyl)-7-(diethylamino)coumarin zu gamma-Cyclodextrin von 1:10000 erhalten wird. Die so erhaltene Farbstoffkomplexlösung wird anschließend in ein Reaktionsgefäß transferiert und bis zur Trockenen lyophilisiert.Das Lyophilisat besitzt eine Finalkonzentration von 1µM an Coumarin-6.

Zu 10ml einer Tumorzellen enthaltenden Probe werden 1.1mg des Lyophilisats zugesetzt. Nach einer Inkubation bei Umgebungstemperatur während 10min werden die Proben spektroskopisch bzw. Fluoreszenzmikroskopisch analysiert und ausgewertet.

### Beispiel 5 (Färbung von Tumorzellen mittels Lyophilisat - Coumarin-120 und beta-Cyclodextrin)

100mg beta-Cyclodextrin (Sigma-Aldrich, Österreich) werden im Ultraschallbad unter Erwärmen auf 65°C in 10ml Wasser gelöst.

Zu 10ml dieser Lösung werden unter Rühren 0,5ml einer 1:1000 Verdünnung (in Ethanol) von 17,5mg 3-(2-Benzothiazolyl)-7-(diethylamino)coumarin in 5ml Ethanol zugefügt, sodass ein Molverhältnis von 7-Amino-4-methylcoumarin zu beta-Cyclodextrin von 1:10000 erhalten wird. Die so erhaltene Farbstoffkomplexlösung wird anschließend in ein Reaktionsgefäß transferiert und bis zur Trockenen lyophilisiert. Das Lyophilisat besitzt eine Finalkonzentration von 1µM an Coumarin.

Zu 10ml einer Tumorzellen enthaltenden Probe werden 1mg des Lyophilisats zugesetzt. Nach einer Inkubation bei Umgebungstemperatur während 10min werden die Proben spektroskopisch bzw. Fluoreszenzmikroskopisch analysiert und ausgewertet.

### Literaturverzeichnis

1. Creaven, P.J., D.V. Parke, and R.T. Williams, A Spectrofluorimetric Study of 7-Hydroxylation of Coumarin by Liver Microsomes. Biochemical Journal, 1965. 96(2): p. 390-&.
2. Kim, H.S. and I.W. Wainer, Rapid analysis of the interactions between drugs and human serum albumin (HSA) using high-performance affinity chromatography (HPAC). Journal of Chromatography B-Analytical Technologies in the Biomedical and Life Sciences, 2008. 870(1): p. 22-26.
3. Liu, X.H., et al., Spectroscopic studies on binding of 1-phenyl-3-(coumarin-6-yl)sulfonylurea to bovine serum albumin. Journal of Photochemistry and Photobiology B-Biology, 2008. 92(2): p. 98-102.
4. Pelkonen, O. and H. Raunio, Metabolic activation of toxins: Tissue-specific expression and metabolism in target organs. Environmental Health Perspectives, 1997. 105: p. 767-774.
5. Prabu, P., et al., In vitro evaluation of poly(caporlactone) grafted dextran (PGD) nanoparticles with cancer cell. Journal of Materials Science-Materials in Medicine, 2008. 19(5): p. 2157-2163.
6. Kovalenko, S.N., Stepanian, S.G., Chuev, V.P., Asimov, M.M., Nikitchenko, V.M. and Chernykh, V.P., Modelling of Formation Processes of Inclusion Complexes of Coumarin Laser Dyes and ß-Cyclodextrin by the MM2 Force Field Method.I. 7-Amino-4-methyl-Coumarins Mol. Eng., 1992. 2: p. 153-163.
7. Velic, D., Knapp, M and Köhler, G, Supramolecular inclusion complexes between a coumarin dye and β-cyclodextrin, and attachment kinetics of thiolated β-cyclodextrin to gold surface. J. Mol. Struct., 2001. 598: p. 49-56.
8. Vyas, A., S. Saraf, and S. Saraf, Cyclodextrin based novel drug delivery systems. Journal of Inclusion Phenomena and Macrocyclic Chemistry, 2008. 62(1-2): p. 23-42.
9. Hirayama, F. and K. Uekama, Cyclodextrin-based controlled drug release system. Advanced Drug Delivery Reviews, 1999. 36(1): p. 125-141.
10. Dougherty, T.J. and S.L. Marcus, Photodynamic Therapy. European Journal of Cancer, 1992. 28A(10): p. 1734-1742.
11. Vanhillegersberg, R., W.J. Kort, and J.H.P. Wilson, Current Status of Photodynamic Therapy in Oncology. Drugs, 1994. 48(4): p. 510-527.
12. Lacy, A.a.O.K., R., Studies on Coumarins and Coumarin-Related Compounds to Determine their Therapeutic Role in the Treatment of Cancer. Current Pharmacetical Design, 2004. 10(30): p. 3797-811.
13. Frank, S.G., Inclusion Compounds. J. Pharm. Sci., 1975. 64: p. 1585-604.
14. Fery-Forgues , S., R. El-Ayoubi, and J. Lamere, F., Fluorescent Microcrystals Obtained from Coumarin 6 using the Reprecipitation Method. J Fluorescence, 2008. 18: p. 619-624.
15. Hicks, R.M. and J.S.J. Wakefield, Membrane Changes during Urothelial Hyperplasia and Neoplasia. Cancer Research, 1976. 36(7): p. 2502-2507.

## Patentansprüche

1. Verfahren zur qualitativen und/oder quantitativen Bestimmung von Tumorzellen, umfassend die Schritte:
a) Erzeugen eines Farbstoffkomplexes aus einem Aminocoumarin und Cyclodextrin;
b) Versetzen der Tumorzellen mit dem in Schritt a) hergestellten Farbstoffkomplex;
c) Inkubation der Zellen mit dem in Schritt a) hergestellten Farbstoffkomplex;
d) Bestimmen der Tumorzellen mittels Fluoreszenzmikroskopie und/oder fluoreszensspektrometrischer Analyse.

2. Verfahren nach Anspruch 1, **dadurch gekennzeichnet, dass** das Aminocoumarin aus der Gruppe, umfassend Coumarin-6, Coumarin-30 (auch Coumarin-515), Coumarin-35, Coumarin-47, Coumarin-102 (auch Coumarin-480), Coumarin-120, Coumarin-138, Coumarin-151, Coumarin-152, Coumarin-153, Coumarin-500, ausgewählt wird und bevorzugt Coumarin-6 ist.

3. Verfahren nach Anspruch 1 oder 2, **dadurch gekennzeichnet, dass** das Cyclodextrin aus der Gruppe umfassend alpha-Cyclodextrin, beta-Cyclodextrin oder gamma-Cyclodextrin ausgewählt wird und bevorzugt beta- und/oder gamma-Cyclodextrin ist.

4. Verfahren nach einem der Ansprüche 1 bis 3, **dadurch gekennzeichnet, dass** das molare Verhältnis von Aminocoumarin und Cyclodextrin von 1:2000 bis 1:20000 reicht und bevorzugt 1:10000 beträgt.

5. Verfahren nach einem der Ansprüche 1 bis 4, **dadurch gekennzeichnet, dass** der Farbstoffkomplex in gelöster und/oder lyophilisierter Form eingesetzt wird.

6. Verfahren nach einem der Ansprüche 1 bis 5, **dadurch gekennzeichnet, dass** der Verfahrensschritt c) in einem Temperaturbereich von 5°C bis 45°C, bevorzugt bei Umgebungstemperatur von 18°C bis 22°C, insbesondere bevorzugt bei 20°C, oder Körpertemperatur von 35°C bis 39°C, insbesondere bevorzugt bei 37°C erfolgt.

7. Verfahren nach einem der Ansprüche 1 bis 6, **dadurch gekennzeichnet, dass** die Tumorzellen in Körperflüssigkeiten vorliegen.

8. Verfahren nach einem der Ansprüche 1 bis 7, **dadurch gekennzeichnet, dass** die Tumorzellen nach dem Verfahrensschritt c) und vor der fluoreszenzmikroskopischen und/oder fluoreszensspektrometrischen Analyse einem Abtrennungsschritt unterzogen werden.

9. Verwendung eines Farbstoffkomplexes aus Aminocoumarin und Cyclodextrin zur Markierung von Tumorzellen.

10. Verwendung nach Anspruch 9, **dadurch gekennzeichnet, dass** das Aminocoumarin aus der Gruppe, umfassend Coumarin-6, Coumarin-47, Coumarin-120, Coumarin-138, Coumarin-151, Coumarin-152, Coumarin-153 ausgewählt wird und bevorzugt Coumarin-6 ist.

11. Verwendung nach Anspruch 9 oder 10, **dadurch gekennzeichnet, dass** das Cyclodextrin aus der Gruppe umfassend alpha-Cyclodextrin, beta-Cyclodextrin oder gamma-Cyclodextrin ausgewählt wird und bevorzugt beta- und/oder gamma-Cyclodextrin ist.

12. Verwendung nach einem der Ansprüche 9 bis 11, **dadurch gekennzeichnet, dass** das molare Verhältnis von Aminocoumarin und Cyclodextrin von 1:2000 bis 1:20000 reicht und bevorzugt 1:10000 beträgt.

## Claims

1. A method for qualitative and/or quantitative analysis of tumor cells, comprising the following steps:
a) generating a dye complex from an aminocoumarin and cyclodextrin;
b) mixing the tumor cells with the dye complex prepared in step a);
c) incubating the cells with the dye complex prepared in step a);
d) analyzing the tumor cells by means of fluorescence microscopy and/or fluorescence spectrometric analysis.

2. The method as defined by claim 1, **characterized in that** the aminocoumarin is selected from the group comprising coumarin 6, coumarin 30 (also coumarin 515), coumarin 35, coumarin 47, coumarin 102 (also coumarin 480), coumarin 120, coumarin 138, coumarin 151, coumarin 152, coumarin 153, coumarin 500, and is preferably coumarin 6.

3. The method as defined by claim 1 or 2, **characterized in that** the cyclodextrin is selected from the group comprising alpha-cyclodextrin, beta-cyclodextrin, or gamma-cyclodextrin, and is preferably beta- and/or gamma-cyclodextrin.

4. The method as defined by one of claims 1-3, **characterized in that** the molar ratio of aminocoumarin and cyclodextrin ranges from 1:2,000 to 1:20,000 and is preferably 1:10,000.

5. The method as defined by one of claims 1-4, **characterized in that** the dye complex is used in dissolved and/or lyophilized form.

6. The method as defined by one of claims 1-5, **characterized in that** method step c) takes place in a temperature range of 5°C to 45°C, preferably at ambient temperature of 18°C to 22°C, especially preferably at 20°C, or at body temperature of 35°C to 39°C, especially preferably at 37°C.

7. The method as defined by one of claims 1-6, **characterized in that** the tumor cells are present in body fluids.

8. The method as defined by one of claims 1-7, **characterized in that** the tumor cells, after method step c) and before the fluorescence microscopic analysis and/or fluorescence spectrometric analysis, are subjected to a separation step.

9. Use of a dye complex comprising aminocoumarin and cyclodextrin for marking tumor cells.

10. The use as defined by claim 9, **characterized in that** the aminocoumarin is selected from the group comprising coumarin 6, coumarin 47, coumarin 120, coumarin 138, coumarin 151, coumarin 152, coumarin 153, and is preferably coumarin 6.

11. The use as defined by claim 9 or 10, **characterized in that** the cyclodextin is selected from the group comprising alpha-cyclodextrin, beta-cyclodextrin, or gamma-cyclodextrin, and is preferably beta- and/or gamma-cyclodextrin.

12. The use as defined by one of claims 9-11, **characterized in that** the molar ratio of aminocoumarin and cyclodextrin ranges from 1:2,000 to 1:20,000 and is preferably 1:10,000.

## Revendications

1. Procédé de détermination qualitative et/ou quantitative de cellules tumorales, comprenant les étapes suivantes :
a) production d'un complexe colorant composé d'une aminocoumarine et de cyclodextrine ;
b) mélange des cellules tumorales avec le complexe colorant produit dans l'étape a) ;
c) incubation des cellules avec le complexe colorant produit dans l'étape a) ;
d) détermination des cellules tumorales au moyen de la microscopie à fluorescence et/ou de l'analyse par spectrométrie de fluorescence.

2. Procédé selon la revendication 1, **caractérisé en ce que** l'aminocoumarine est sélectionnée dans le groupe comprenant coumarine-6, coumarine-30 (également coumarine-515), coumarine-35, coumarine-47, coumarine-102 (également coumarine-480), coumarine-120, coumarine-138, coumarine-151, coumarine-152, coumarine-153, coumarine-500, et est de préférence coumarine-6.

3. Procédé selon la revendication 1 ou 2, **caractérisé en ce que** la cyclodextrine est sélectionnée dans le groupe comprenant alpha-cyclodextrine, bêta-cyclodextrine ou gamma-cyclodextrine et est de préférence bêta-cyclodextrine et/ou gamma-cyclodextrine.

4. Procédé selon une des revendications 1 à 3, **caractérisé en ce que** le rapport molaire de l'aminocoumarine et de la cyclodextrine s'étend de 1:2000 à 1:20000 et est de préférence égal à 1:10000.

5. Procédé selon une des revendications 1 à 4, **caractérisé en ce que** le complexe colorant est mis en oeuvre sous forme dissoute et/ou lyophilisée.

6. Procédé selon une des revendications 1 à 5, **caractérisé en ce que** l'étape de procédé c) s'effectue dans une plage de température de 5 °C à 45 °C, de préférence à la température ambiante de 18 °C à 22 °C, de façon particulièrement préférée à 20 °C, ou à la température corporelle de 35 °C à 39 °C, de façon particulièrement préférée à 37 °C.

7. Procédé selon une des revendications 1 à 6, **caractérisé en ce que** les cellules tumorales sont présentes dans des fluides corporels.

8. Procédé selon une des revendications 1 à 7, **caractérisé en ce que**, après l'étape de procédé c) et avant l'analyse par microscopie à fluorescence et/ou par spectrométrie de fluorescence, les cellules tumorales sont soumises à une étape de séparation.

9. Utilisation d'un complexe colorant en aminocoumarine et en cyclodextrine pour le marquage de cellules tumorales.

10. Utilisation selon la revendication 9, **caractérisée en ce que** l'aminocoumarine est sélectionnée dans le groupe comprenant coumarine-6, coumarine-47, coumarine-120, coumarine-138, coumarine-151, coumarine-152, coumarine-153, et est de préférence coumarine-6.

11. Utilisation selon la revendication 9 ou 10, **caractérisée en ce que** la cyclodextrine est sélectionnée dans le groupe comprenant alpha-cyclodextrine, bêta-cyclodextrine ou gamma-cyclodextrine et est de préférence bêta-cyclodextrine et/ou gamma-cyclodextrine.

12. Utilisation selon une des revendications 9 à 11, **caractérisée en ce que** le rapport molaire de l'aminocoumarine et de la cyclodextrine s'étend de 1:2000 à 1:20000 et est de préférence égal à 1:10000.
